# EUROPEAN PATENT APPLICATION

(11) **EP 4 286 526 A1**
(43) Date of publication of application: **06.12.2023**
(21) Application number: 22746043.3
(22) Date of filing: 28.01.2022
(51) Int. Cl.: C12Q 1/06, C12Q 1/6869, G01N 33/48, G06Q 50/10

(54) **METHOD FOR PREDICTING OBESITY**

(30) Priority: 29.01.2021 JP 2021012817; 30.07.2021 JP 2021126318
(71) Applicant: Anicom Holdings, Inc., Shinjuku-ku Tokyo 160-0023 (JP)
(72) Inventor: MATSUMOTO Kengo, Tokyo 160-0023 (JP); SUMI Hiroshi, Tokyo 160-0023 (JP); TAKAHASHI Hiroyuki, Tokyo 160-0023 (JP); HORIE Ryo, Tokyo 160-0023 (JP)
(74) Representative: Gill Jennings & Every LLP
(86) International application number: PCT/JP2022/003385
(87) International publication number: WO 2022/163824

(57) **Abstract**

An object of the invention is to provide a method for predicting and determining obesity in an animal, which is a method for predicting obesity in an animal, including a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae that are present in the body of the animal or in a sample isolated from the animal.

## Description

### TECHNICAL FIELD

The present invention relates to a method for predicting obesity in an animal, a method for determining obesity in an animal, and a method for predicting disease in an animal, and specifically relates to a method for predicting obesity for predicting whether an animal will become obese based on the measurement of the number of bacterial species in the body of an animal or in a sample obtained from the animal.

### BACKGROUND ART

Pet animals such as dogs, cats, and rabbits, as well as livestock such as cows and pigs, are irreplaceable to humans. In recent years, while the average lifespan of animals raised by humans has increased significantly, animals are more likely to suffer from some kind of disease during their lifetime, and the increase in medical expenses borne by animal owners has become a problem.

Especially in recent years, the problem of obesity in animals due to lack of exercise, excessive intake of food, genetic factors, or the like has become apparent in the same way as humans. It has been pointed out that obesity can cause various diseases, and is considered to be one of the causes of increased medical expenses for animals.

Meanwhile, pet insurance is offered to cover medical expenses for pet animals. As with life insurance and medical insurance for humans, pet insurance premiums and whether or not pet insurance can be purchased are often determined according to the breed, age, and medical history of an animal. Needless to say, for pet insurance policyholders, it is preferable that the animals covered by insurance are healthy.

Therefore, it is desired to provide pet insurance policyholders with information on the health of animals covered by insurance to increase their motivation to maintain the health of the animals.

Obesity in animals is partly due to genetic factors, but most of it is thought to be caused by lifestyle habits. It is expected that the provision of information and enlightenment to pet owners and pet insurance policyholders will increase the possibility of preventing and eliminating obesity in animals. Therefore, there is a demand for a method that can provide information on animal obesity and related diseases in a simple manner.

Patent Document 1 discloses a gut microbiota-regulating or -improving composition having an effect of effectively regulating or improving gut microbiota by increasing bacteria of the phylum Bacteroidetes and reducing bacteria of the phylum Firmicutes in the gut microbiota, but does not disclose the relationship between obesity and specific bacterial species in animal bodies (animal intestines) or in samples taken from animals, such as the gut microbiota.

### RELATED ART DOCUMENT(S)

### PATENT DOCUMENT(S)

[Patent Document 1] WO 2017/094892

### SUMMARY OF THE INVENTION

### PROBLEMS TO BE SOLVED BY THE INVENTION

Accordingly, an object of the present invention is to provide a method for predicting and determining obesity in an animal.

### MEANS FOR SOLVING THE PROBLEMS

The present inventors analyzed and examined a large amount of data on the obesity state of animals covered by pet insurance and the state of the microbiota in the bodies of the animals. As a result, the inventors found a relationship between obesity and a specific bacterial group in animal bodies or in samples taken from animals, such as the gut microbiota. This has led to the completion of the present invention.

Specifically, the present invention is the following [1] to [22].
[1] A method for predicting obesity in an animal, including a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.
[2] The method for predicting obesity in an animal according to [1], wherein the body of the animal refers to an intestine.
[3] The method for predicting obesity in an animal according to [1], wherein the sample isolated from the animal is animal feces.
[4] The method for predicting obesity in an animal according to any one of [1] to [3], wherein the measurement step is to measure the number of bacterial species belonging to the families Coriobacteriaceae and/or Prevotellaceae.
[5] The method for predicting obesity in an animal according to any one of [1] to [4], further including a measurement step of measuring an occupancy rate of bacteria belonging to one or more families selected from the group consisting of Alcaligenaceae, Fusobacteriaceae, and Veillonellaceae.
[6] The method for predicting obesity in an animal according to any one of [1] to [5], wherein the animal is a dog.
[7] The method for predicting obesity in an animal according to any one of [1] to [6], wherein the age of the animal is 5 years old or younger.
[8] A method for determining obesity in an animal, including a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.
[9] A method for predicting disease morbidity of an animal, including a measurement step of measuring an occupancy rate of bacteria belonging to one or more families selected from the group consisting of Coriobacteriaceae, Prevotellaceae, Coprobacillaceae, Paraprevotellaceae, Alcaligenaceae, Fusobacteriaceae, and Veillonellaceae present in the body of the animal or in a sample isolated from the animal.
[10] A method for calculating an animal insurance premium, including calculating an insurance premium based on a prediction result of the method for predicting obesity in an animal according to any one of [1] to [7].
[11] A method for predicting obesity in an animal, including a step of measuring a diversity index of the gut microbiota of the animal.
[12] The method for predicting obesity in an animal according to [11], wherein the diversity index is the Shannon-Wiener diversity index.
[13] A method for predicting obesity in an animal, including a step of predicting whether or not the animal will become obese from data on the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae present in the body of the animal or in a sample isolated from the animal.
[14] A method for predicting obesity in an animal, including a step of predicting whether or not the animal will become obese from data on an occupancy rate of bacteria belonging to one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae present in the body of the animal or in a sample isolated from the animal.
[15] The method for predicting obesity in an animal according to [14], wherein the data on the occupancy rate of bacteria is a score given according to the occupancy rate.
[16] A system for predicting obesity in an animal, including: reception means for receiving data on the number of bacterial species or occupancy rate of bacteria belonging to a predetermined bacterial family regarding bacteria present in the body of an animal or in a sample isolated from the animal; and determination means for predicting whether or not the animal will become obese from data on the number of bacterial species or occupancy rate of bacteria belonging to a predetermined bacterial family regarding bacteria present in the body of an animal or in a sample isolated from the animal.
[17] The system for predicting obesity in an animal according to [16], further including a display unit for displaying a prediction result by the determination means using an icon corresponding to the susceptibility to obesity.
[18] The system for predicting obesity in an animal according to [17], further including a display unit for displaying the number of bacterial species or a degree of the occupancy rate of bacteria belonging to a predetermined bacterial family using an icon, together with the prediction result by the determination means.
[19] The system for predicting obesity in an animal according to any one of [16] to [18], further including advice means for providing advice for improving obesity according to the prediction result by the determination means.
[20] The system for predicting obesity in an animal according to any one of [16] to [19], further including proposal means for recommending food according to the prediction result by the determination means.
[21] The system for predicting obesity in an animal according to any one of [16] to [20], wherein the predetermined bacterial family is one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae.
[22] The system for predicting obesity in an animal according to any one of [16] to [20], wherein the predetermined bacterial family is one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae.

### EFFECTS OF THE INVENTION

The present invention allows providing a method for predicting and determining obesity in an animal.

### BRIEF DESCRIPTION OF THE DRAWINGS

FIG. 1 is a graph diagram showing the results of Examples.
FIG. 2A is a graph diagram showing the results of Examples.
FIG. 2B is a graph diagram showing the results of Examples.
FIG. 3 is a graph diagram showing the results of Examples.
FIG. 4 is a graph diagram showing the results of Examples.
FIG. 5 is a graph diagram showing the results of Examples.
FIG. 6 is a graph diagram showing the results of Examples.
FIG. 7 is an example of icons displayed by the display unit of the present invention.
FIG. 8 is an example of icons displayed by the display unit of the present invention.
FIG. 9 is a schematic diagram showing one embodiment of the animal obesity prediction system of the present invention.
FIG. 10 is a schematic diagram showing one embodiment of the animal obesity prediction system of the present invention.
FIG. 11 is a graph diagram showing the results of Examples.

### MODE FOR CARRYING OUT THE INVENTION

### <Method for Predicting Obesity>

One method for predicting obesity according to the present invention is characterized by including a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.

### [Target Animals]

A target animal is not particularly limited and is preferably a mammal, such as a dog, cat, rabbit, or ferret, and a dog is particularly preferred.

The age of the animal is preferably 5 years old or younger, particularly preferably 3 to 5 years old.

### [Sample]

Any known method can be used for examining and measuring the type and number of bacteria present in the body of an animal or in a sample isolated from an animal. The bacteria are preferably intestinal bacteria, and the sample is preferably animal feces. In other words, in a preferred embodiment, animal feces is used as a sample to examine the animal's gut microbiota.

### [Measurement of Number of Bacterial Species]

The number of bacterial species can be measured using a known metagenomic analysis method such as amplicon sequencing using a sequencer such as NGS, or a microbiota analysis method. For example, there is a method of identifying an organism contained in a sample by analyzing DNA or RNA base sequence information of all organisms contained in the sample using a next-generation sequencer. Preferably, there is a method for obtaining composition data of bacteria in a sample by amplifying all or part of the 16S rRNA gene contained in the sample as necessary to perform sequencing and analyzing the obtained sequence using the software. By processing the composition data of bacteria in the sample with software or referring to genetic databases such as Genbank, Greengenes, and SILVA database, it is possible to determine the attribution of the species of bacteria contained in the sample and measure the number of species of bacteria belonging to a specific family.

An example of 16S rRNA gene amplicon analysis (microbiota analysis) using NGS (next-generation sequencer) will be specifically described. First, DNA is extracted from a sample using a DNA extraction reagent, and the 16S rRNA gene is amplified from the extracted DNA by PCR. Then, the amplified DNA fragments are comprehensively sequenced using NGS, and after removing low-quality reads and chimeric sequences, the sequences are clustered, and operational taxonomic unit (OTU) analysis is performed. OTU is an operational classification unit for treating sequences having a certain degree of similarity or more (for example, 96% to 97% or more homology) as if they were a single bacterial species. It is therefore considered that the number of OTUs represents the number of bacterial species that constitute the microbiota, and the number of reads belonging to the same OTU represents the relative abundance of that species. In addition, it is possible to select a representative sequence from among the number of reads belonging to each OTU and to identify the family name and genus species name by database search. In this way, the number of bacterial species belonging to a specific family can be measured.

### [Prediction Method]

The method for predicting obesity of the present invention is characterized by including a measurement step of measuring the number of bacterial species belonging to a specific family that is present in the body of an animal or in a sample isolated from the animal. When the number of bacterial species is few, it is predicted that the animal will become obese in the future. The expression "in the future" used herein means "within a predetermined period" and the period is not particularly limited, but preferably within 3 years, more preferably within 2 years, and still more preferably within 1 year. Moreover, since it is a prediction of obesity, it is fully conceivable that the animal will not develop obesity by improving the diet and lifestyle habits of the animal after using the method of the present invention. Instead, it can also be used to encourage the pet owner of the animal to improve the diet and lifestyle habits of the animal by telling the pet owner that the animal is at risk of becoming obese. For example, according to the prediction result of the method for predicting obesity of the present invention, meals for preventing obesity, supplements containing bacteria that are unlikely to cause obesity, low-calorie meals, low-carbohydrate meals, diet menus, and the like can be proposed and recommended.

In addition, according to the prediction result of the method for predicting obesity of the present invention, beverages, meals, and supplements for preventing obesity can be produced or customized. A service related to obesity prevention can be in the form of prediction by the method for predicting obesity of the present invention, provision of the prediction result, production or customization of beverages, meals, and supplements according to the prediction result, and proposal and recommendation of the beverages, meals, and supplements. Moreover, after providing such a service, it is also possible to implement the method for predicting obesity of the present invention and present whether or not the obesity tendency has improved. The beverages, meals, and supplements described above include dietary beverages, diet foods, dietary supplement additives, and the like.

In addition, it is preferable to predict obesity by combining the results of measuring the number of bacterial species with the results of genetic testing (presence or absence of genes that are likely to affect obesity). By combining the results of genetic testing, obesity can be predicted with high accuracy.

As a criterion for obesity, for example, with regard to the body condition score (BCS) of each animal (breed), it can be said that BCS is four or more on a five-point scale (six or more on a nine-point scale), but obesity is not limited to this criterion. In an animal for which the BCS is not specified, for example, a body fat percentage can be used as a reference. In this case, obesity means that the body fat percentage of the animal is higher than the average value of the animal species, preferably 50% or more, more preferably 75% or more, but obesity is not limited thereto. In addition, regarding an animal for which the BCS is not specified, obesity can be defined by referring to a dog or cat for which the BCS is specified.

As a result of verification by the present inventors, it was found that obese or obese-prone animals have fewer species of bacteria belonging to the family Streptococcaceae, Ruminococcaceae, Bifidobacteriaceae, Enterococcaceae, Erysipelotrichaceae, Coriobacteriaceae, Prevotellaceae, Coprobacillaceae, or Paraprevotellaceae in their gut microbiota than non-obese animals. Therefore, it can be understood that the number of bacterial species of these bacterial families is related to obesity, and that when the number of bacterial species of these bacterial families is few or has decreased, obesity is likely to occur. On the other hand, when the number of these bacterial species is many or has increased, it can be predicted and determined that obesity is unlikely to occur.

A decrease in the number of bacterial species or a small number of bacterial species may be determined based on a comparison with the number of bacterial species in an animal of the same species that is not obese or not prone to obesity or may be determined by measuring the number of bacterial species in the same animal a plurality of times over time and observing changes in the number of bacterial species in the animal.

It is also possible to construct a database of the number of bacterial species belonging to the above-described family for each animal species and compare the number of bacterial species with that in the database, thereby confirming that the number of bacterial species has decreased or is few.

For example, when the number of bacterial species is preferably 1% or more, more preferably 3% or more, and still more preferably 5% or more compared to that in a target animal or the average value of target animals, it can be predicted that the animal is likely to become obese.

In another example, the number of bacterial species in the same animal is preferably 1% or more, more preferably 3% or more, and still more preferably 5% or more compared to that at the time of the previous measurement. It can be predicted that the animal is likely to become obese.

In addition, the method for predicting obesity of the present invention may be a method for predicting obesity in an animal, including a step of predicting whether or not the animal will become obese from data on the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae present in the body of the animal or in a sample isolated from the animal.

Data on the number of bacterial species include, for example, the number of bacterial species, a score given according to the number of bacterial species, and a score given according to whether the number of bacterial species is greater than or equal to a predetermined value or less than a predetermined value. A total score calculated by summing the scores of each bacterial family may be used.

In addition, the method for predicting obesity of the present invention may be a method for predicting obesity in an animal, including a step of predicting whether or not the animal will become obese from data on an occupancy rate of bacteria belonging to one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae present in the body of the animal or in a sample isolated from the animal.

Data on the occupancy rate of bacteria include, for example, the occupancy rate, a score given according to the occupancy rate, and a score given according to whether the occupancy rate is greater than or equal to a predetermined value or less than a predetermined value. A total score calculated by summing the scores of each bacterial family may be used.

### [Family to be measured]

The family targeted for measurement is one or more selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae. It is particularly preferable to measure the number of bacterial species belonging to the families Coriobacteriaceae and/or Prevotellaceae. The present inventors confirmed that the number of bacterial species belonging to these families was reduced in both obese animals and animals affected with obesity-related diseases, compared to healthy animals.

In addition to the above, or instead of the above, the occupancy rate of bacteria belonging to one or more families selected from the group consisting of Alcaligenaceae, Fusobacteriaceae, and Veillonellaceae may be measured. The occupancy rate refers to the abundance ratio (detection ratio) of each bacterial species in the microbiota such as the gut microbiota.

In addition to the above, or instead of the above, the families Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae can be included as the target families to be measured.

### [Streptococcaceae]

The family Streptococcaceae is placed within the order Lactobacillales and includes the genera Lactococcus, Lactovum, and Streptococcus.

### [Ruminococcaceae]

Ruminococcaceae is a family of bacteria belonging to the order Clostridiales of the class Clostridia. In addition to the genus Ruminococcus, the genera Acetanaerobacterium, Acutalibacter, Anaerobacterium, Anaerofilum, Anaerotruncus, Dysosmobacter, Ercella, Ethanoligenens, Faecalibacterium, Fastidiosipila, Hydrogenoanaerobacterium, Oscillibacter, Oscillospira, Papillibacter, Pseudobacteroides, Sporobacter, Subdoligranulum, and Youngiibacter are included.

### [Bifidobacteriaceae]

Bifidobacteriaceae is a family within the order Bifidobacteriales and includes the genus Bifidobacterium.

### [Enterococcaceae]

Enterococcaceae is a family of Gram-positive eubacteria belonging to the order Lactobacillales. Examples of representative genera thereof include the genera Enterococcus, Melissococcus, Pilibacter, Tetragenococcus, and Vagococcus.

### [Erysipelotrichaceae]

Erysipelotrichaceae is a family belonging to the order Erysipelotrichales, and includes the genera Allobaculum, Breznakia, Bulleidia, Catenibacterium, Catenisphaera, Coprobacillus, Dielma, Dubosiella, Eggerthia, Erysipelothrix, Faecalibaculum, Holdemania, Ileibacterium, Kandleria, Longibaculum, Longicatena, Solobacterium, and Turicibacter.

### [Coriobacteriaceae]

Coriobacteriaceae is a family belonging to the order Coriobacteriales, and includes the genera Atopobium and Olsenella.

### [Prevotellaceae]

Prevotellaceae is a family belonging to the order Bacteroidales, and includes the genus Prevotella.

### [Coprobacillaceae]

Coprobacillaceae is a family belonging to the order Erysipelotrichales, and includes the genus Coprobacillus.

### [Paraprevotellaceae]

The family Paraprevotellaceae is a family belonging to the order Bacteroidales, and includes the genus Paraprevotella. In addition, there is also a taxonomy that the genus Paraprevotella is included in the family Prevotellaceae, without defining the family Paraprevotellaceae as an independent family.

### [Alcaligenaceae]

Alcaligenaceae is a family belonging to the order Burkholderiales, and includes the genus Alcaligenes.

### [Fusobacteriaceae]

Fusobacteriaceae is a family belonging to the order Fusobacteriales, and includes the genus Fusobacterium.

### [Veillonellaceae]

Veillonellaceae is a family belonging to the order Clostridiales and includes the genera Acetonema, Acidaminococcus, Allisonella, Anaeroarcus, Anaeroglobus, Anaeromusa, Anaerosinus, Anaerospora, Anaerovibrio, Centipeda, Dendrosporobacter, Desulfosporomusa, Dialister, Megamonas, Megasphaera, Mitsuokella, Pectinatus, Pelosinus, Phascolarctobacterium, Propionispira, Propionispora, Psychrosinus, Quinella, Schwartzia, Selenomonas, Sporomusa, Sporotalea, Succiniclasticum, Succinispira, Thermosinus, Veillonella, and Zymophilus.

### [Bacteroidaceae]

Bacteroidaceae is a family belonging to the order Bacteroidales of the class Bacteroidia of the phylum Bacteroidota, and includes the genus Bacteroides.

### [Porphyromonadaceae]

Porphyromonadaceae is a family belonging to the order Bacteroidales, and includes the genus Porphyromonas.

### [Erysipelatoclostridiaceae]

Erysipelatoclostridiaceae is a family belonging to the order Erysipelotrichales, and includes the genus Erysipelatoclostridium.

### [Gemellaceae]

Gemellaceae is a family belonging to the order Staphylococcus, and includes the genus Gemella.

### [Clostridiaceae]

Clostridiaceae is a family belonging to the order Clostridiales, and includes the genus Clostridium.

### [Oscillospiraceae]

Oscillospiraceae is a family belonging to the order Eubacteriales, and includes the genus Oscillospira.

### [Acidaminococcaceae]

Acidaminococcaceae is a family belonging to the order Acidaminococcales, and includes the genus Acidaminococcus.

### [Selenomonadaceae]

Selenomonadaceae is a family belonging to the order Selenomonadales, and includes the genus Selenomonas.

### [Saccharimonadaceae]

Saccharimonadaceae is a family belonging to the order Saccharimonadales, and includes the genus Saccharimonas.

### [Sphingomonadaceae]

Sphingomonadaceae is a family of the order Sphingomonadales, and includes the genus Sphingomonas.

### [Helicobacteraceae]

Helicobacteraceae is a family belonging to the order Campylobacterales of the class Epsilonproteobacteria, and includes the genus Helicobacter.

### [Desulfovibrionaceae]

Desulfovibrionaceae is a family belonging to the order Desulfovibrionales of the class Deltaproteobacteria, and includes the genus Desulfovibrio.

### [Succinivibrionaceae]

Succinivibrionaceae is a family belonging to the order Aeromonadales of the class Gammaproteobacteria, and includes the genera Aeromonas and Anaerobiospirillum.

### [Sutterellaceae]

Sutterellaceae is a family of the order Burkholderiales, and includes the genus Sutterella.

### [Morganellaceae]

Morganellaceae is a family of the order Enterobacterales, and includes the genus Morganella.

### <Method for Determining Obesity>

The method for determining obesity in an animal according to the present invention includes a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.

For the one or more families, when the number of bacterial species belonging to the family has decreased or is few, it can be determined that the animal is obese.

A decrease in the number of bacterial species or a small number of bacterial species may be determined based on a comparison with the number of bacterial species in an animal of the same species that is not obese or not prone to obesity or may be determined by measuring the number of bacterial species in the same animal a plurality of times over time and observing changes in the number of bacterial species in the animal.

It is also possible to construct a database of the number of bacterial species belonging to the above-described family for each animal species and compare the number of bacterial species with that in the database, thereby confirming that the number of bacterial species has decreased or is few.

For example, when the number of bacterial species in a target animal is preferably 1% or more, more preferably 3% or more, and still more preferably 5% or more compared to that in a control animal of the normal body size or the average value of control animals (average value of animals that their pet owners answered that the body shape is "normal" and animals that have the standard body shape), it can be predicted that the animal is likely to become obese.

In another example, in a case where the number of bacterial species in the same animal is preferably 1% or more, more preferably 3% or more, and still more preferably 5% or more compared to that at the time of the previous measurement, it can be predicted that the animal is likely to become obese.

### <Method for Predicting Disease Morbidity>

The method for predicting disease morbidity of the present invention is characterized by including a measurement step of measuring an occupancy rate of bacteria belonging to one or more families selected from the group consisting of Coriobacteriaceae, Prevotellaceae, Coprobacillaceae, Paraprevotellaceae, Alcaligenaceae, Fusobacteriaceae, and Veillonellaceae present in the body of an animal or in a sample isolated from the animal.

Studies by the present inventors revealed that the occupancy rate and bacteria-carrying rate of bacteria belonging to the family Coriobacteriaceae, Prevotellaceae, Coprobacillaceae, Paraprevotellaceae, Alcaligenaceae, Fusobacteriaceae, or Veillonellaceae in the gut microbiota are low in animals affected with a certain disease with a high prevalence rate in animals of the obese group (endocrine disease, respiratory disease, blood disease, musculoskeletal disease, skin disease, ear disease, systemic disease, liver/biliary/pancreatic disease). It is therefore understood that bacteria belonging to these families are associated with the prevalence rate of a certain disease associated with obesity.

For the one or more families, when the number of bacterial species or occupancy rate of the bacterial species belonging to the family has decreased or is few, it can be determined that the animal is (highly) likely to be affected by the disease.

A decrease in the number of bacterial species or a small number of bacterial species may be determined based on a comparison with the number of bacterial species in an animal of the same species or may be determined by measuring the number of bacterial species in the same animal a plurality of times over time and observing changes in the number of bacterial species in the animal.

It is also possible to construct a database of the number of bacterial species belonging to the above-described family for each animal species and compare the number of bacterial species with that in the database, thereby confirming that the number of bacterial species has decreased or is few.

For example, when the number of bacterial species is preferably 1% or more, more preferably 3% or more, and still more preferably 5% or more compared to that in a target animal or the average value of target animals, it can be predicted that the animal is likely to be affected by the disease.

In another example, in a case where the number of bacterial species in the same animal is preferably 1% or more, more preferably 3% or more, and still more preferably 5% or more compared to that at the time of the previous measurement, it can be predicted that the animal is likely to be affected by the disease.

### [Method for Calculating Insurance Premium]

A method for calculating an insurance premium according to the present invention is characterized by calculating the insurance premium based on prediction results obtained by the method for predicting obesity in an animal. Since it is assumed that animals that are expected to become obese, those that are likely to become obese, or those that have a tendency to become obese are more likely to be affected by diseases, especially diseases related to obesity, in the future compared to animals of the same species with a normal body type, insurance premiums can be calculated to be higher. In addition, for example, for animals that are not expected to have a tendency to become obese, those that are unlikely to become obese, or those that do not have a tendency to become obese, insurance premiums can be calculated at low prices.

### [Diversity Index]

The method for predicting obesity of the present invention is a method for predicting obesity in an animal which includes a step of measuring a diversity index of the gut microbiota of the animal. Examples of the diversity index include the Shannon-Wiener diversity index (Shannon index), the Simpson diversity index, and the like, and the Shannon-Wiener diversity index is preferred.

It can be predicted that the higher the diversity index of the gut microbiota of an animal, the less likely the animal will become obese.

The diversity index of the gut microbiota can be measured by a known method. Other points are the same as above.

### <System for Predicting Obesity in Animals>

The system for predicting obesity in an animal of the present invention includes: reception means for receiving data on the number of bacterial species or occupancy rate of bacteria belonging to a predetermined bacterial family regarding bacteria present in the body of an animal or in a sample isolated from the animal; and determination means for predicting whether or not the animal will become obese from data on the number of bacterial species or occupancy rate of bacteria belonging to a predetermined bacterial family regarding bacteria present in the body of an animal or in a sample isolated from the animal.

### [Reception Means]

The reception means for the present invention is means for receiving input of data on the number of bacterial species or occupancy rate of bacteria belonging to a predetermined bacterial family in an animal-derived sample for which the possibility of obesity is to be predicted. Examples of animals include dogs, cats, birds, rabbits, and ferrets. In addition, although the age of a target animal is not limited, it is preferably 0 years old or older, more preferably 0 to 12 years old, and still more preferably 1 to 10 years old. The method of receiving data on the number of bacterial species or occupancy rate may be any method such as inputting or transmitting data to a terminal.

The data on the number of bacterial species or occupancy rate are data related to the number of bacterial species or occupancy rate of each bacterium contained in an animal-derived sample such as a stool sample or in the gut microbiota. The occupancy rate is the abundance ratio (detection ratio) of bacteria belonging to each bacterial family in the microbiota in the sample. For example, it can be measured as a "hit rate" as a result of detection by a known metagenomic analysis method such as amplicon sequencing using a sequencer such as NGS. In the present invention, as the data on the number of bacterial species or occupancy rate, the number of bacterial species or occupancy rate of the microbiota may be used, and labels and scores set based on the number of bacterial species or occupancy rate may be used.

The occupancy rate in the present invention is the occupancy rate for each bacterial family. The occupancy rate for each family is the occupancy rate for all bacteria belonging to a certain family. In other words, when calculating the occupancy rate for each family, the occupancy rate of the family can be calculated by summing the occupancy rate of the bacterial species belonging to a certain family for each bacterial species in the microbiota. It may be summed by the family with identification at the species or genus level, or identification at the family level may be performed to calculate the occupancy rate of the family without identification at the species or genus level.

A label set based on the occupancy rate is a label appropriately set according to the magnitude of the numerical value of the occupancy rate. For example, according to the numerical value of the occupancy rate, the following three levels of labels can be set: "large," "medium," and "small" or "many," " medium," and "few." Also, the number of levels of labels can be arbitrarily set, and for example, multilevel labels such as "0," "1," "2," "3," ..., "20" can be given.

When a label is used, the occupancy rate in the microbiota is measured, and before entering the number into the input means, a specific label is assigned from a predetermined correspondence table according to the measured occupancy rate such that the label can be entered into the reception means.

In addition, the label set based on the number of bacterial species is a label appropriately set according to the number of bacterial species. For example, a label such as "1" can be given when the number of bacterial species exceeds a predetermined value, and "0" when it does not.

A score set based on the occupancy rate is a score appropriately set according to the magnitude of the numerical value of the occupancy rate. For example, a score of "+1" is given when the occupancy rate is equal to or higher than a certain reference value, and "-1" is given when it is less than a certain reference value.

In addition, the score set based on the number of bacterial species is a score appropriately set according to the number of bacterial species. For example, for a specific family, a score of "+1" is given when the number of bacterial species belonging to that family is greater than or equal to a predetermined value, and a score of "0" or "-1" is given when it is less than or equal to a predetermined value.

Such a score may be calculated for each bacterial family and input to the reception means. It is also possible to use a configuration in which the number of bacterial species and the occupancy rate of bacteria belonging to the bacterial family is input into the reception means and a score is calculated for each bacterial family based on the score assignment criteria set in advance based on the input data on the number of bacterial species or occupancy rate.

The system for predicting obesity of the present invention may be configured such that the determination means sums the scores calculated or input for each bacterial family, and predicts and determines the possibility of becoming obese based on the obtained total score.

### [Determination Means]

The determination means of the present invention is means for predicting and determining whether or not an animal will become obese within a predetermined period from data on the number of bacterial species or occupancy rate of bacteria belonging to a predetermined bacterial family in a sample derived from the animal, the data being input into the reception means. A method for prediction and determination is not particularly limited. For example, a processor predicts and determines whether or not an animal will become obese within a predetermined period from data on the number of bacterial species or occupancy rate of bacteria belonging to a predetermined bacterial family in a sample derived from the animal. As described above, it is also possible to use a configuration in which a score is calculated according to preset criteria from the number of bacterial species or occupancy rate for each bacterial family, and the risk of obesity is determined based on the total score obtained by summing the scores. In other words, when the total score is equal to or greater than a predetermined value, the risk of obesity is high (or the risk of obesity is low), and when the total score is less than the predetermined value, the risk of obesity is low (or the risk of obesity is high).

When data on the number of bacterial species or occupancy rate of bacteria belonging to a predetermined bacterial family in the microbiota in a sample derived from an animal are received, the determination means of the present invention predicts and determines whether or not the animal will become obese within a predetermined period of time, preferably a predetermined period of time from the time of reception, the time of obtaining the sample, or the time of obtaining data on the number of bacterial species or occupancy rate of bacteria belonging to a predetermined bacterial family in the microbiota. The predetermined period is preferably within 3 years, more preferably within 2 years, and still more preferably within 1 year.

The determination means of the present invention may be configured to predict and determine using a learned model. Such a learned model is preferably a learned model that has learned the relationship between data on the number of bacterial species or occupancy rate of bacteria belonging to a predetermined bacterial family in the microbiota and information as to whether or not the animal became obese within a specified time period from the time of obtaining a sample or the time of obtaining data on the number of bacterial species or occupancy rate of bacteria belonging to a predetermined bacterial family in the microbiota. Further, the learned model is preferably a learned model that has learned, as training data, data on the number of bacterial species or occupancy rate of bacteria belonging to a predetermined bacterial family in the microbiota and information as to whether or not the animal became obese within a specified time period from the time of obtaining a sample or the time of obtaining data on the number of bacterial species or occupancy rate of bacteria belonging to a predetermined bacterial family in the gut microbiota. The predetermined period in the information as to whether or not the animal became obese within a specified time period used as training data is preferably within 3 years, more preferably within 2 years, and still more preferably within 1 year.

Artificial intelligence (AI) is preferable as the learned model. Artificial intelligence (AI) is software or a system that mimics the intellectual work performed by the human brain with a computer, which is specifically a computer program that understands natural human language, makes logical inferences, and learns from experience, or the like. Artificial intelligence may be of a general-purpose or specialized type and may be a deep neural network, a convolutional neural network, or the like, and open software can be used.

In order to generate a learned model, AI is allowed to learn using training data. Learning may be either machine learning or deep learning, but machine learning is preferred. Deep learning is a development of machine learning and is characterized by automatically finding a feature quantity. In the present invention, for example, data on the number of bacterial species or occupancy rate of bacteria belonging to a predetermined bacterial family in the gut microbiota can be used as a feature quantity.

A learning method for generating a learned model is not particularly limited, and publicly available software can be used. For example, the deep learning GPU training system (DIGITS) published by NVIDIA can be used. In addition, for example, learning may be performed by a known support vector machine method or the like published in "An Introduction to Support Vector Machines and Other Kernel-based Learning Methods" (KYORITSU SHUPPAN CO., LTD.) or the like.

Machine learning can be either unsupervised learning or supervised learning, but supervised learning is preferred. The method of supervised learning is not particularly limited, and examples thereof include decision tree, ensemble learning, and gradient boosting. Examples of published machine learning algorithms include XGBoost, CatBoost, and LightGBM.

Training data for learning include, for example, data on the number of bacterial species or occupancy rate of bacteria belonging to a predetermined bacterial family in the microbiota in a sample derived from an animal, and the presence or absence of obesity which means whether or not the animal became obese within a predetermined period, preferably within 3 years, more preferably within 2 years, still more preferably within 1 year, and particularly preferably within 180 days from the time of obtaining the sample (e.g., fecal sample) or the time of obtaining data on the number of bacterial species or occupancy rate of bacteria belonging to a predetermined bacterial family in the microbiota in the sample. Whether or not the animal became obese can be replaced with a dummy variable. The data on the number of bacterial species or occupancy rate of bacteria belonging to a predetermined bacterial family in the microbiota in a sample derived from an animal used as training data are the same as data on the number of bacterial species or occupancy rate of bacteria belonging to a predetermined bacterial family in the microbiota in a sample derived from an animal as described for the method of receiving data above. Information as to whether the animal became obese within a predetermined period can be obtained from the owner or the like who provided the sample from the animal using, for example, a smartphone application or questionnaire.

### [Output]

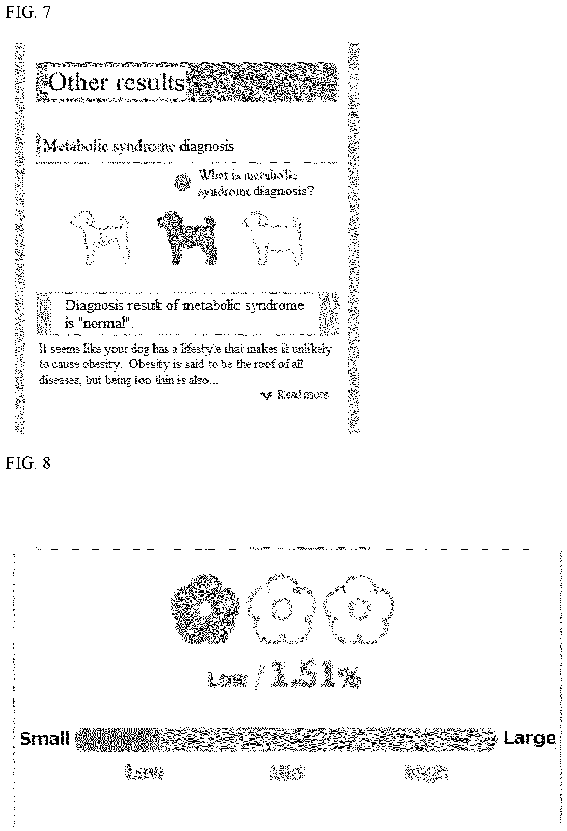

The output format of prediction and determination by the determination means of the present invention is not particularly limited. For example, the prediction and determination can be output by displaying "likely to become obese within the next year," "highly likely to become obese within the next year," "likely to become obese within the next year with a probability of *% (* indicates the number calculated)," "prone to become obese," or the like on the screen of a terminal such as a personal computer or a smartphone. It is preferable to display an icon on the screen of the user's terminal to indicate whether the animal is prone to become obese. For example, as shown in FIG. 7, it is preferable that the icon represents the susceptibility to obesity in several stages graphically such that the user can easily understand.

The system for predicting obesity in an animal of the present invention may additionally have output means for receiving the determination result from the determination means and outputting the determination result.

It is preferable that the system for predicting obesity in an animal of the present invention has a display unit that displays the prediction result obtained by the determination means as well as whether the number of bacterial species or occupancy rate of bacteria belonging to a predetermined bacterial family is large or small, for example, using icons as shown in FIG. 8.

It is preferable that the system for predicting obesity in an animal of the present invention further includes advice means for advising a lifestyle improvement method according to the result of obesity prediction. For example, the advice means receives the prediction result output from the determination means and, according to the prediction result, proposes to increase the number and distance of walks, to go to a dog run, or to adjust the life rhythm. The advice means may have a learned model.

It is preferable that the system for predicting obesity in an animal of the present invention further includes proposal means for proposing (recommending) food according to the result of predicting obesity. For example, the proposal means can receive the prediction result output from the determination means and, according to the prediction result, propose or recommend food for avoiding the predicted risk of obesity, such as supplements containing bacteria that are unlikely to cause obesity, low-salt meals, low-calorie meals, low-sugar meals, diet menus, and the like. The proposal means may have a learned model.

### [Preferred Family]

As described above, the system for predicting obesity in an animal of the present invention uses data on the number of bacterial species or occupancy rate of bacteria belonging to a predetermined bacterial family. The predetermined bacterial family is not particularly limited, but examples thereof include, for example, the above-described families. In addition, the bacterial family is preferably one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae.

In addition, the predetermined bacterial family is preferably one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae.

The system for predicting obesity in an animal of the present invention may use data on the number of bacterial species or occupancy rate of bacteria belonging to a predetermined bacterial family in the microbiota in a sample derived from an animal as well as information such as face image, type, breed, age, sex, weight, medical history, gene sequence information, SNP, presence or absence of gene mutation, and the like.

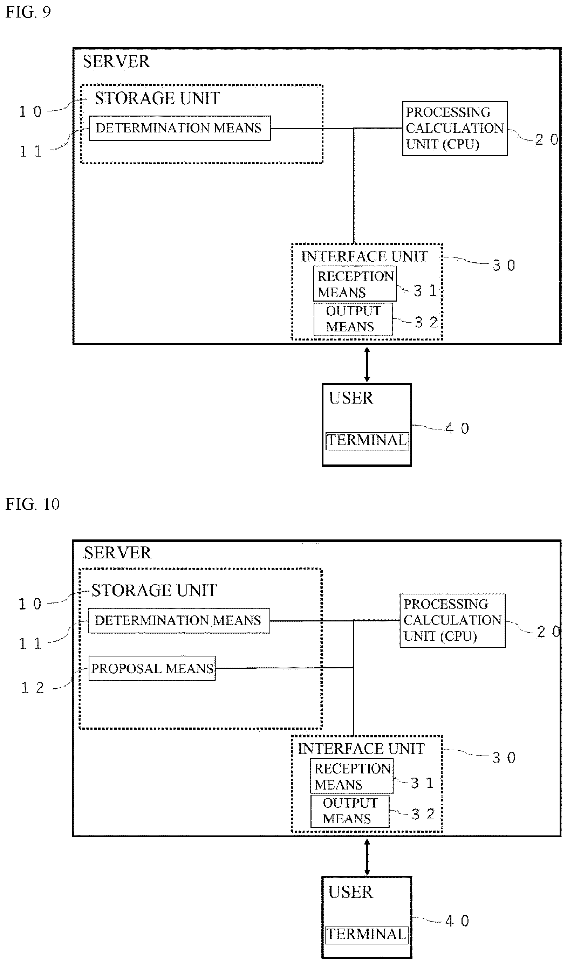

One embodiment of the system for predicting obesity in an animal of the present invention will be described below with reference to FIG. 9.

In FIG. 9, a terminal 40 is used by a person (user) who wants to use the system for predicting obesity. Examples of the terminal 40 include a personal computer, a smartphone, and a tablet terminal. The terminal 40 is configured to include a processing unit such as a CPU, a storage unit such as a hard disk, ROM, or RAM, a display unit such as a liquid crystal panel, an input unit such as a mouse, keyboard, or touch panel, a communication unit such as a network adapter, and the like.

The user accesses the server from the terminal 40 and inputs and transmits data on the number of bacterial species or occupancy rate of bacteria belonging to a predetermined bacterial family in the microbiota, preferably gut microbiota (hereinafter, explained in the examples of gut microbiota) in a sample of a target animal and, if necessary, information such as face image (photograph), type, breed, age, weight, medical history, and the like of the animal.

In addition, the user can receive a result of predicting obesity from the server by accessing the server with the terminal 40.

In addition, the user receives a fecal sample collection kit for examining the gut microbiota of the animal being raised and sends the fecal sample to a company that measures the gut microbiota (not shown). The company measures the gut microbiota of the animal and obtains data on the number of bacterial species or occupancy rate of bacteria belonging to a predetermined bacterial family gut microbiota. Those skilled in the art may directly input and transmit data on the number of bacterial species or occupancy rate of bacteria belonging to a predetermined bacterial family in the gut microbiota of the animal to reception means 31 of the server via its own terminal. Alternatively, those skilled in the art may separately send data on the number of bacterial species or occupancy rate of bacteria belonging to a predetermined bacterial family in the gut microbiota of the animal (pet) to the user by postal mail, e-mail, or the like such that the user can input and transmit data on the number of bacterial species, the occupancy rate, or diversity in the gut microbiota to the reception means 31 through the terminal 40.

In this embodiment, the server is configured to include a computer, but any device may be used as long as it has the functions of the present invention.

A storage unit 10 is configured to include, for example, a ROM, a RAM, or a hard disk. The storage unit 10 stores an information processing program for operating each unit of the server, particularly software for a determination means 11, and the like.

The determination means 11 inputs data on the number of bacterial species or occupancy rate of bacteria belonging to a predetermined bacterial family in the gut microbiota of a target animal input by a user or a company which measured the gut microbiota as described above, and outputs a prediction as to whether the animal will become obese within a predetermined period (for example, within one year), or the probability (%) of becoming obese. A learned model may be used as the determination means. Such a learned model is configured to include, for example, XGBoost, CatBoost, LightGBM, or a deep neural network or convolutional neural network.

In this embodiment, an aspect in which determination means and reception means are stored in a server and connected to a user's terminal via connection means such as the Internet or LAN has been explained. However, the present invention is limited thereto. An aspect in which determination means, the reception means, and an interface unit are stored in one server or device, an aspect that does not require a separate terminal for the user to use, or the like is possible.

The system for predicting obesity in an animal of the present invention may include proposal means 12 (the same applies to the advice means) as shown in FIG. 10. The proposal means 12 is software that selects and proposes food suitable for an animal based on the obesity prediction output by the determination means 11 and information entered by the user, such as the type, breed, age at the time of obtaining gut microbiota data, weight, medical history, and the like of the animal. For example, the software is software that grades obesity tendency according to the type, breed, and age at the time of obtaining gut microbiota data, weight, medical history, and the like of the animal, and finally corrects the grade in consideration of the obesity prediction output by the determination means so as to recommend food suitable for obesity measures based on the obesity tendency grade

The same software may include the proposal means 12 and the determination means 11.

A processing calculation unit 20 uses the determination means 11 and the proposal unit 12 stored in the storage unit so as to calculate an obesity prediction and food recommendation result.

An interface unit (communication unit) 30 includes the reception means 31 and output means 32, receives data on the number of bacterial species or occupancy rate of bacteria belonging to a predetermined bacterial family in the gut microbiota of an animal and other information from the user's terminal, and outputs the obesity prediction and the food recommendation result to the user's terminal.

### EXAMPLES

### [Example 1]

### (DNA Extraction from Stool Sample)

A fecal sample was collected from each of dogs (toy poodle) and DNA was extracted in the following manner.

A dog (toy poodle) owner collected a fecal sample from a dog using a fecal collection kit. The fecal sample was received and suspended in water.

Next, 200 µL of the fecal suspension and 810 µL of lysis buffer (containing 224 µg/mL ProteinaseK) were added to a bead tube, and beads were crushed with a bead homogenizer (6,000 rpm, 20 seconds of crushing, 30 seconds of interval, 20 seconds of crushing). Subsequently, the sample was left to stand on a heat block at 70°C for 10 minutes to be treated with ProteinaseK, and left to stand on a heat block at 95°C for 5 minutes so as to inactivate ProteinaseK. Using chemagic (trademark) 360 (PerkinElmer), DNA was automatically extracted from the lysed specimen according to the chemagic kit stool protocol, thereby obtaining 100 µL of DNA extract.

### (Meta Sequence Analysis of 16S RNA Gene)

16S metasequence analysis was performed with modified illumina 16SMetagenomic Sequencing Library Preparation (version 15044223B). First, a 460-bp region containing the variable regions V3-V4 of the 16S rRNA gene was amplified by PCR using universal primers (Illumina_16S_341F and Illumina_16S_805RPCR). The PCR reaction solution was prepared by mixing 10 µL of the DNA extract, 0.05 µL of each primer (100 µM), 12.5 µL of 2x KAPA HiFi Hot-Start ReadyMix (F. Hoffmann-La Roche, Switzerland), and 2.4 µL of PCR-grade water. For PCR, after heat denaturation at 95°C for 3 minutes, a cycle of 95°C for 30 seconds, 55°C for 30 seconds, and 72°C for 30 seconds was repeated 30 times, and finally, an extension reaction was performed at 72°C for 5 minutes. An amplified product was purified using magnetic beads and eluted with 50 µL of Buffer EB (QIAGEN, Germany). PCR was performed on the amplified product after purification using the Nextera (trademark) XT Index Kitv2 (illumina, CA, US) and indexed. A PCR reaction solution was prepared by mixing 2.5 µL of the amplified product, 2.5 µL of each primer, 12.5 µL of 2x KAPA HiFi (trademark) Hot-Start ReadyMix, and 5 µL of PCR-grade water. For PCR, after heat denaturation at 95°C for 3 minutes, a cycle of 95°C for 30 seconds, 55°C for 30 seconds, and 72°C for 30 seconds was repeated 12 times, and finally, an extension reaction was performed at 72°C for 5 minutes. The amplified product that was indexed was purified using magnetic beads and eluted with 80 to 105 µL of Buffer EB. The concentration of each amplified product was measured with a NanoPhotometer (Implen, CA, US) and adjusted to 1.4 nM. Then, the amplified products were mixed in equal amounts, thereby preparing a library for sequencing. The DNA concentration of the sequencing library and the size of the amplified product were confirmed by electrophoresis and analyzed by MiSeq. For analysis, MiSeq Reagent Kit V3 was used so as to perform paired-end sequencing of 2 × 300 bp. The obtained sequences were analyzed with MiSeq Reporter, thereby obtaining bacterial composition data.

The sequences of the universal primers used above are as follows. Those commercially available can be purchased as universal primers.
Illumina_16S_341F
   5'-TCGTCGGCAGCGTCAGATGTGTATAAGAGACAGCCTACGGGNGGCWGCAG-3'
llumina_16S_805R

5'-
GTCTCGTGGGCTCGGAGATGTGTATAAGAGACAGGACTACHVGGGTATCTAATCC
-3'

According to the above-described method, the number of bacterial species in each family in the gut microbiota was measured for 9390 individuals of toy poodles aged 3 to 13 (including 1029 obese individuals). The classification of whether or not each individual was obese was based on medical interview data on the physique of the individual from the pet owner. For the medical interview data, the pet owner was asked to choose from three options regarding the physique of a target dog: "overweight," "underweight," and "normal." The medical interview data were answered by the insured persons of pet insurance, though based on their subjective judgment as the pet owners. Since the pet owners have no motive to lie, they are deemed to be in constant contact with their dogs and know the information about the dogs, and they are less likely to give a wrong answer because only three easy-to-understand choices are presented, it is considered that the data are reliable to some extent.

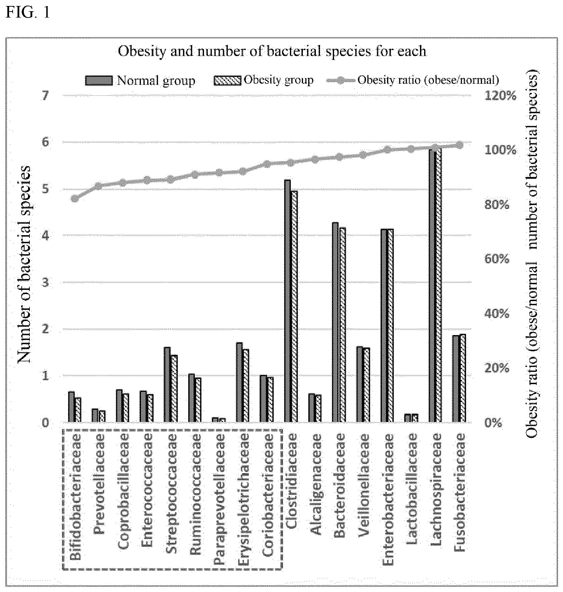

FIG. 1 shows the results of measuring the number of bacterial species. In FIG. 1, the number of bacterial species indicated by the bar graph is the average of bacterial species detected in each bacterial family in the normal group and the obese group. The average may be less than 1 because there are individuals in which the species is not detected.

As is apparent from FIG. 1, it was revealed that the number of bacterial species in toy poodles belonging to the obese group was fewer than that of toy poodles belonging to the normal group by 5% or more for each of the families Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae. The rate of decrease in the number of bacterial species (%) was calculated as "average number of bacterial species in the obese group/average number of bacterial species in the normal group."

### (Calculation of Diversity Index (Shanonn Index))

From the obtained bacterial composition data (FASTQ file), the Shannon index (Shannon-Winner diversity index) was calculated using QIIME2. Then, the individuals were divided into a 3- to 5-year-old group and a 6- to 11-year-old group, and the correlation between the Shanon index and the obesity rate was examined.

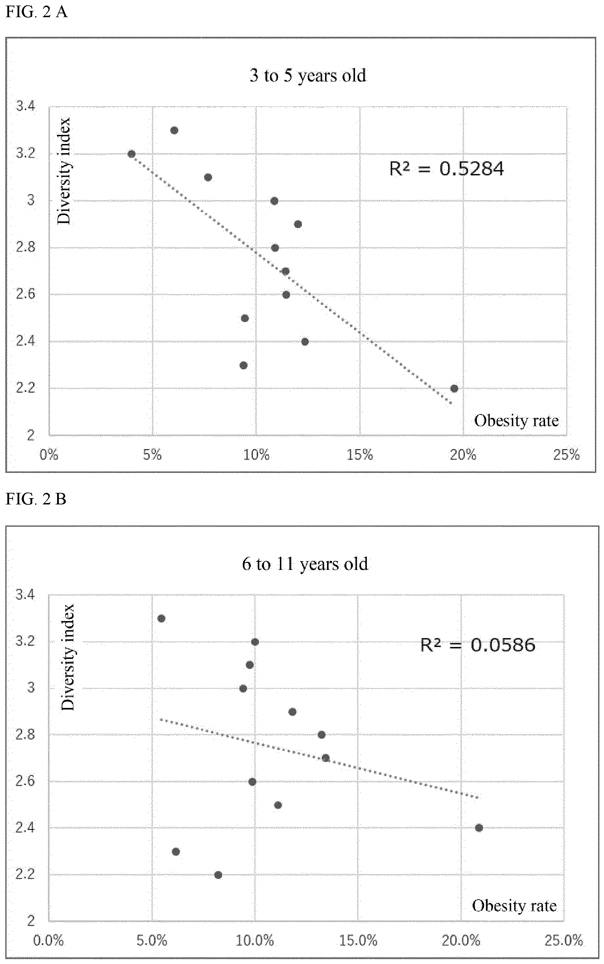

The results are shown in FIGS. 2A and 2B.

As is apparent from FIGS. 2A and 2B, it was found that there is a high correlation between the diversity index (Shanon Index) and the obesity rate in the 3- to 5-year-old group. The term "obesity rate" as used herein refers to the rate of obese individuals in a group of individuals indicating the same diversity index. At that time, the diversity index was rounded to the second decimal place and aligned to the first place. For example, in a case where there are 120 individuals detected to have a diversity index of "3.2" and 12 of them are obese, the obesity rate with a diversity index of 3.2 is 10%.

### (Prevalence Rates of Obesity and Disease)

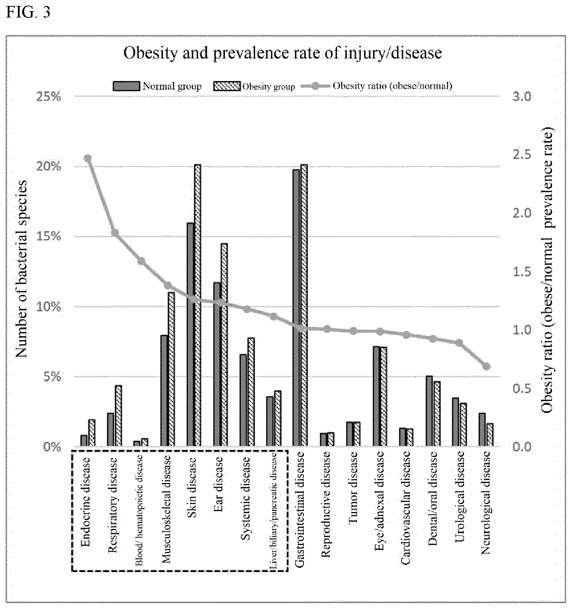

For the toy poodles belonging to the obese group and the toy poodles belonging to the normal group, a graph was made showing whether they were affected by a disease within 180 days before and after the medical interview response, respectively. The results are shown in FIG. 3. The presence or absence of the disease was investigated by using pet insurance claim records to confirm whether or not insurance claims were made within 180 days before and after the date of stool collection for each animal from which stool was collected.

As is apparent from FIG. 3, it is understood that toy poodles belonging to the obese group have high prevalence rates of diseases such as endocrine disease, respiratory disease, blood disease, musculoskeletal disease, skin disease, ear disease, systemic disease, liver/biliary/pancreatic disease (hereinafter referred to as "specific diseases").

### (Prevalence of Specific Diseases and Number of Bacterial Species)

The occupancy rate and bacteria-carrying rate of bacterial species belonging to specific families in the gut microbiota were measured for toy poodles affected with specific diseases (3 to 13 years old; 3,199 individuals in total) and toy poodles without such diseases (3 to 13 years old; accident-free group; 5,088 individuals).

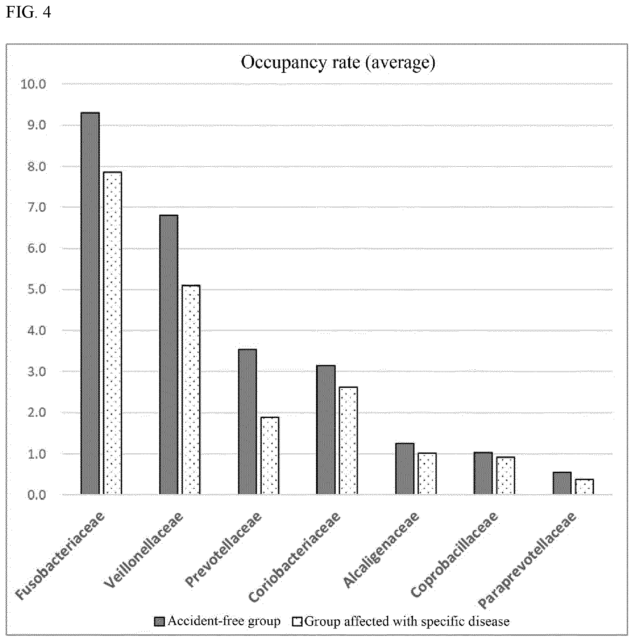

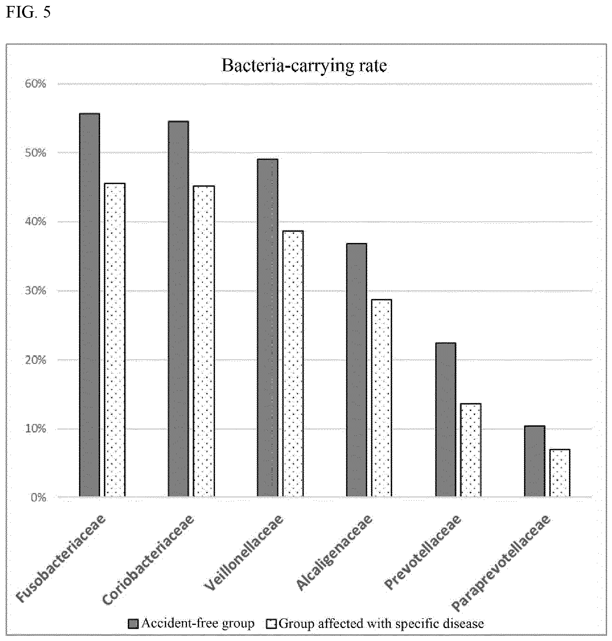

The results are shown in FIGS. 4 and 5. FIG. 4 is a graph showing the occupancy rate of bacterial species belonging to a specific family (the occupancy rate is the abundance ratio (detection ratio) of each bacterial species in the gut microbiota, and is synonymous with "hit rate" in the detection results). FIG. 5 is a graph showing the bacteria-carrying rate (the bacteria-carrying rate is the proportion of individuals having a bacterium in the entire group in a specific category such as the normal group, the obese group, or the like; the bacteria-carrying rate is a value related only to whether each individual carries or does not carry the bacterium and does not include quantitative magnitude relationships such as "to what extent" in terms of "hit rate" or the like).

It is understood from FIGS. 4 and 5 that prevalence of a specific disease is associated with the presence or absence of the possession of bacterial species belonging to the family Coriobacteriaceae, Prevotellaceae, Coprobacillaceae, Paraprevotellaceae, Alcaligenaceae, Fusobacteriaceae, or Veillonellaceae.

### (Correlation between Number of Bacterial Species and Obesity Rate)

For 9,390 individuals of toy poodles aged 3 to 13 (of which 1,029 individuals were obese) surveyed to calculate the data in FIG. 1, the total number of bacterial species of the families Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae and the obesity rate were plotted and graphed. The term "obesity rate" as used herein refers to the rate of obese individuals in a population indicating the same number of bacterial species.

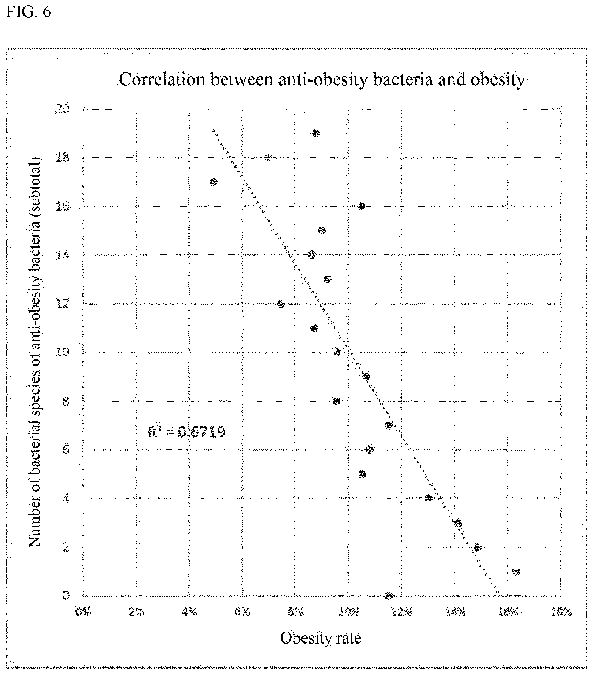

The results are shown in FIG. 6.

It is understood from FIG. 6 that there is a strong correlation between the number of bacterial species belonging to the above-described families and obesity.

### [Example 2]

Fecal samples from dogs were collected in the same manner as in Example 1, and the number of bacterial species per family in the gut microbiota was measured for 3,610 individual dogs aged 0 to 18 years. Obesity was classified into three groups of obese, normal, and underweight according to medical interview data on the physique of the dog from the pet owners. There were 677 individuals in the obese group, 2,671 individuals in the normal group, and 262 individuals in the underweight group.

By comparing the obese group and the normal group in terms of the occupancy rate (hit rate) at the family level obtained from the medical interview, the bacterial families in which there was a difference in the occupancy rate between the obese group and the normal group were selected. The families Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae were selected as such bacterial families.

Each of the 3,610 dogs was scored according to the values of occupancy rates of these bacterial families as follows.
Bifidobacteriaceae: If the hit rate is 0, the score is -1, otherwise 2.
Coriobacteriaceae: If the hit rate is 0, the score is -1, otherwise 1.
Bacteroidaceae: If the hit rate is more than 0.3, the score is -1; if 0, then 0, otherwise 1.
Porphyromonadaceae: If the hit rate is 0, the score is 0, otherwise 1.
Prevotellaceae: If the hit rate is 0, the score is -1; if more than 0.05, then 2, otherwise 0.
Erysipelatoclostridiaceae: If the hit rate is more than 0.01, the score is 2, otherwise -2.
Erysipelotrichaceae: If the hit rate is more than 0.01, the score is 2, otherwise -2.
Gemellaceae: If the hit rate is 0, the score is 0, otherwise -2.
Clostridiaceae: If the hit rate is more than 0, the score is 1; if more than 0.01, then -1, otherwise 0.
Oscillospiraceae: If the hit rate is 0, the score is -1, otherwise 1.
Ruminococcaceae: If the hit rate is 0, the score is -2; if more than 0.003, then 2, otherwise -1.
Acidaminococcaceae: If the hit rate is 0, the score is 0, otherwise 1.
Selenomonadaceae: If the hit rate is 0, the score is -1, otherwise 1.
Fusobacteriaceae: If the hit rate is 0, the score is 0; if more than 0.02, then 0, otherwise 2.
Saccharimonadaceae: If the hit rate is 0, the score is 0, otherwise 2.
Sphingomonadacea: If the hit rate is 0, the score is 0, otherwise -2.
Helicobacteraceae: If the hit rate is 0, the score is 0, otherwise 2.
Desulfovibrionaceae: If the hit rate is 0, the score is 0, otherwise 1.
Succinivibrionaceae: If the hit rate is 0, the score is 0, otherwise 2.
Sutterellaceae: If the hit rate is 0, the score is -1, otherwise 1.
Morganellaceae: If the hit rate is 0, the score is 0, otherwise -2.

A total score was calculated by summing the above-described scores for each individual.

Each individual was classified according to the following categories based on the value of the total score.
-4 or less: "Prone to be overweight"
-3 to 4: "Average"
5 to 25: "Hard to be overweight"

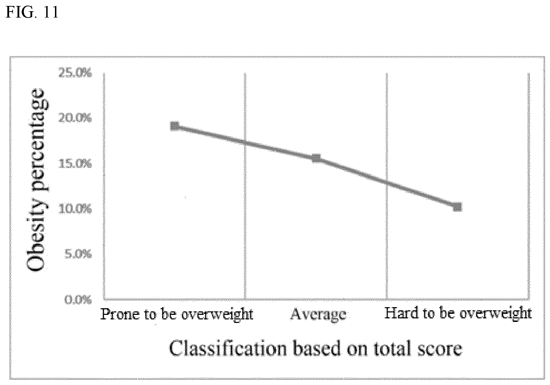

Based on the value of the total score above, the proportion of individuals with the medical interview response above was "obese" (referred to as "obesity percentage") was calculated for the individuals classified into each group. Table 1 shows the relationship between the classification based on the total score and the classification based on the medical interview (obesity percentage). In addition, FIG. 11 shows a graph representing the values in Table 1.

**[Table 1]**

| Classification based on total score | Obesity percentage |
|---|---|
| Prone to be overweight | 19.1% |
| Average | 15.5% |
| Hard to be overweight | 10.2% |

As is apparent from Table 1 and FIG. 11, there is a correlation between the classification based on the total score calculated from the data on the occupancy rate of the bacterial family and the obesity percentage. For example, the group classified as "Prone to be overweight" from the total score has a high obesity percentage.

## Claims

1. A method for predicting obesity in an animal, comprising a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.

2. The method for predicting obesity in an animal according to claim 1, wherein the body of the animal refers to an intestine.

3. The method for predicting obesity in an animal according to claim 1, wherein the sample isolated from the animal is animal feces.

4. The method for predicting obesity in an animal according to any one of claims 1 to 3, wherein the measurement step is to measure the number of bacterial species belonging to the families Coriobacteriaceae and/or Prevotellaceae.

5. The method for predicting obesity in an animal according to any one of claims 1 to 4, further comprising a measurement step of measuring an occupancy rate of bacteria belonging to one or more families selected from the group consisting of Alcaligenaceae, Fusobacteriaceae, and Veillonellaceae.

6. The method for predicting obesity in an animal according to any one of claims 1 to 5, wherein the animal is a dog.

7. The method for predicting obesity in an animal according to any one of claims 1 to 6, wherein the age of the animal is 5 years old or younger.

8. A method for determining obesity in an animal, comprising a measurement step of measuring the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, and Paraprevotellaceae present in the body of the animal or in a sample isolated from the animal.

9. A method for predicting disease morbidity of an animal, comprising a measurement step of measuring an occupancy rate of bacteria belonging to one or more families selected from the group consisting of Coriobacteriaceae, Prevotellaceae, Coprobacillaceae, Paraprevotellaceae, Alcaligenaceae, Fusobacteriaceae, and Veillonellaceae present in the body of the animal or in a sample isolated from the animal.

10. A method for calculating an animal insurance premium, comprising calculating an insurance premium based on a prediction result of the method for predicting obesity in an animal according to any one of claims 1 to 7.

11. A method for predicting obesity in an animal, comprising a step of measuring a diversity index of the gut microbiota of the animal.

12. The method for predicting obesity in an animal according to claim 11, wherein the diversity index is the Shannon-Wiener diversity index.

13. A method for predicting obesity in an animal, comprising a step of predicting whether or not the animal will become obese from data on the number of bacterial species belonging to one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae present in the body of the animal or in a sample isolated from the animal.

14. A method for predicting obesity in an animal, comprising a step of predicting whether or not the animal will become obese from data on an occupancy rate of bacteria belonging to one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae present in the body of the animal or in a sample isolated from the animal.

15. The method for predicting obesity in an animal according to claim 14, wherein the data on the occupancy rate of bacteria is a score given according to the occupancy rate.

16. A system for predicting obesity in an animal, comprising:
reception means for receiving data on the number of bacterial species or occupancy rate of bacteria belonging to a predetermined bacterial family regarding bacteria present in the body of an animal or in a sample isolated from the animal; and
determination means for predicting whether or not the animal will become obese from data on the number of bacterial species or occupancy rate of bacteria belonging to a predetermined bacterial family regarding bacteria present in the body of an animal or in a sample isolated from the animal.

17. The system for predicting obesity in an animal according to claim 16, further comprising a display unit for displaying the prediction result by the determination means using an icon corresponding to the susceptibility to obesity.

18. The system for predicting obesity in an animal according to claim 17, further comprising a display unit for displaying the number of bacterial species or a degree of the occupancy rate of bacteria belonging to a predetermined bacterial family using an icon, together with the prediction result by the determination means.

19. The system for predicting obesity in an animal according to any one of claims 16 to 18, further comprising advice means for providing advice for improving obesity according to the prediction result by the determination means.

20. The system for predicting obesity in an animal according to any one of claims 16 to 19, further comprising proposal means for recommending food according to the prediction result by the determination means.

21. The system for predicting obesity in an animal according to any one of claims 16 to 20, wherein the predetermined bacterial family is one or more families selected from the group consisting of Streptococcaceae, Ruminococcaceae, Bifidobacteriaceae, Enterococcaceae, Erysipelotrichaceae, Coriobacteriaceae, Prevotellaceae, Coprobacillaceae, Paraprevotellaceae, Alcaligenaceae, Fusobacteriaceae, and Veillonellaceae.

22. The system for predicting obesity in an animal according to any one of claims 16 to 20, wherein the predetermined bacterial family is one or more families selected from the group consisting of Bifidobacteriaceae, Coriobacteriaceae, Bacteroidaceae, Porphyromonadaceae, Prevotellaceae, Erysipelatoclostridiaceae, Erysipelotrichaceae, Gemellaceae, Clostridiaceae, Oscillospiraceae, Ruminococcaceae, Acidaminococcaceae, Selenomonadaceae, Fusobacteriaceae, Saccharimonadaceae, Sphingomonadacea, Helicobacteraceae, Desulfovibrionaceae, Succinivibrionaceae, Sutterellaceae, and Morganellaceae.
